# FASCICULE DE BREVET EUROPEEN

(11) **EP 2 068 744 B1**
(45) Date de publication et mention de la délivrance du brevet: **02.05.2012**
(21) Numéro de dépôt: 07805444.2
(22) Date de dépôt: 04.10.2007
(51) Int. Cl.: A61C 1/08, A61C 5/02

(54) **PIECE A MAIN DENTAIRE A DISPOSITIF DE BUTEE**
DENTALHANDSTÜCK MIT WIDERLAGERVORRICHTUNG
DENTAL HANDPIECE WITH ABUTMENT DEVICE

(30) Priorité: 06.10.2006 FR 0654131
(43) Date de publication de la demande: 17.06.2009
(73) Titulaire: ANTHOGYR, 74700 Sallanches (FR)
(72) Inventeur: RICHARD, Hervé, F-73590 Notre Dame De Bellecombe (FR)
(74) Mandataire: Poncet, Jean-François
(86) Numéro de dépôt international: PCT/IB2007/054045
(87) Numéro de publication internationale: WO 2008/041198

(56) Documents cités:
- DE-B- 1 084 870
- US-B1- 6 213 770
- US-E- R E35 147

## Description

### DOMAINE TECHNIQUE DE L'INVENTION

La présente invention concerne les pièces à main utilisées par les dentistes et chirurgiens dentistes. Plus particulièrement, l'invention concerne une pièce à main dentaire permettant de porter un foret et de l'entraîner en mouvement relatif selon une direction de pénétration afin de réaliser un perçage dans l'os maxillaire ou mandibulaire d'un patient pour y insérer un implant dentaire.

Au cours du perçage, le praticien traverse avec son foret plusieurs zones osseuses de densités différentes. Le praticien doit en effet traverser des zones d'os cortical de surface et des zones d'os spongieux plus en profondeur. L'os cortical étant plus difficile à percer, le praticien est alors amené à appliquer un effort axial important sur la pièce à main dentaire lorsqu'il perce les zones osseuses en surface. Au passage brutal du foret dans une zone d'os spongieux, le praticien ne réduit pas instantanément l'effort axial sur la pièce à main dentaire, et cela peut entraîner le dépassement de la profondeur de forage initialement prévue. Ce dépassement accidentel peut résulter en un sectionnement du nerf dentaire et une désensibilisation partielle ou complète de la zone environnante.

Une première solution pour éviter ce sectionnement accidentel du nerf dentaire a été d'utiliser une pièce à main dentaire munie d'un foret comportant une pluralité de marquages ou gorges annulaires sur sa longueur. Le praticien surveille alors la pénétration du foret par la lecture de ces marquages ou gorges annulaires. Cependant, la présence de sang et/ou de salive au voisinage de la zone de travail du foret empêche une lecture fiable des marquages du foret. Une telle solution n'est donc pas fiable.

Une seconde solution pour éviter ce sectionnement accidentel du nerf dentaire a été d'utiliser des pièces à main dentaires permettant de porter un foret et de l'entraîner en mouvement relatif selon une direction de pénétration, comprenant :
- un corps avec une extrémité proximale et une extrémité distale de corps,
- une tête connectée à l'extrémité distale de corps, apte à tenir le foret,
- des moyens de butée, fixés sur la pièce à main dentaire par des moyens de fixation, et permettant de limiter la profondeur de pénétration du foret dans le corps d'un patient,
dans lesquelles les moyens de butée comprennent un corps de butée, à surface d'appui apte à prendre appui contre le corps du patient en fin de pénétration du foret dans le corps du patient.

Une telle pièce à main est notamment décrite dans le document FR 2 831 050.

L'inconvénient de ces pièces à main est que les moyens de butée fixés sur la pièce à main dentaire présentent un encombrement important susceptible de gêner le praticien lors de son opération. Le praticien ne pourra ainsi pas accéder à certaines zones à traiter de la bouche du patient, dont l'accès est difficile. Cette gêne du praticien peut également résulter en des douleurs inutiles pour le patient, ou en un manque de précision de l'opération chirurgicale ainsi exécutée.

En outre, ces moyens de butée sont difficilement démontables voire impossibles à démonter, et ils gênent ainsi le praticien lors d'autres opérations où celui-ci n'a pas besoin des moyens de butée.

En outre, les moyens de butée de telles pièces à main rendent compliquées les opérations de nettoyage des différents éléments les constituant, par un démontage souvent long et laborieux qui incite le praticien à ne nettoyer parfois que partiellement ou superficiellement les pièces à main dentaires.

Une autre solution pour éviter le sectionnement accidentel du nerf dentaire a été apportée par l'utilisation de forets munis d'un épaulement permettant de faire butée en fin de pénétration du foret dans le corps du patient. L'inconvénient est que de tels forets ne permettent de poser qu'une seule longueur d'implant, leurs moyens de butée étant par nature non réglables. Le praticien devra ainsi avoir recours à un grand nombre de forets pour effectuer des perçages de différentes longueurs. Il en résulte un coût matériel non négligeable pour le praticien, qui voit se multiplier le nombre d'outils nécessaires pour lui permettre de mener à bien son travail, et qui doit changer d'outil plus fréquemment.

L'utilisation de pièces à main dentaires du type de celle du document FR 2 831 050 complique l'usage de forets à épaulement de butée par l'encombrement des moyens de butée qui sont en outre inutiles. Le praticien est ainsi gêné inutilement, ce qui risque de provoquer d'inutiles douleurs chez le patient ou d'entraîner un manque de précision lors de l'opération à exécuter.

Un autre système permettant de fiabiliser les opérations de perçage a été imaginé en utilisant un guide chirurgical muni de canons de perçage et destiné à être utilisé avec des forets dépourvus d'épaulement de butée.

Ce sont les canons de perçage qui servent de butée au foret en fonction de la longueur qui est choisie. L'utilisation d'un guide chirurgical est cependant impossible avec une pièce à main dentaire telle que celle décrite dans le document FR 2 831 050, du fait de l'encombrement des moyens de butée au voisinage de la tête de la pièce à main dentaire.

On connaît également une pièce à main, telle que celle décrite dans le document US 6,213,770, qui comporte des moyens de butée rendus amovibles au moyen d'un engagement réversible tel qu'un encliquetage. Il y a cependant un risque que les moyens de butée se détachent accidentellement et/ou se déplacent par rapport à la pièce à main dentaire pendant son utilisation en bouche. Il en résulte un manque de fiabilité inacceptable pour les praticiens.

### EXPOSE DE L'INTENTION

Un premier problème proposé par la présente invention est de concevoir des moyens de butée fiables, aisément nettoyables, de faible encombrement, aisément et rapidement adaptables sur une pièce à main dentaire pour fiabiliser des opérations de perçage réalisées avec un foret dépourvu d'épaulement de butée, et qui permettent également l'utilisation aisée d'un foret comprenant un épaulement de butée ou l'utilisation d'un guide chirurgical avec des canons de perçage.

Selon un autre aspect, l'invention vise à limiter l'encombrement des moyens de butée, pour ne pas gêner le praticien lorsque celui-ci n'a pas besoin d'utiliser les moyens de butée.

Simultanément, l'invention cherche en outre à concevoir des moyens de butée simples et rapides à monter sur la pièce à main dentaire, ne nécessitant que très peu de modifications de la pièce à main dentaire en vue de les y monter et ne pouvant être fixés sur et détachés de la pièce à main dentaire que lorsque celle-ci n'est pas en cours de fonctionnement.

Pour atteindre ces objets, ainsi que d'autres, l'invention propose une pièce à main dentaire permettant de porter un foret et de l'entraîner en mouvement relatif selon une direction de pénétration, comprenant :
- un corps avec une extrémité proximale et une extrémité distale de corps,
- une tête démontable, connectée à l'extrémité distale de corps, apte à tenir le foret,
- des moyens de butée, fixés sur la pièce à main dentaire par des moyens de fixation, et permettant de limiter la profondeur de pénétration du foret dans le corps d'un patient,
dans laquelle les moyens de butée comprennent :
- un corps de butée, à surface d'appui apte à prendre appui contre le corps du patient en fin de pénétration du foret dans le corps du patient,
- un tronçon de liaison, solidaire du corps de butée et s'étendant selon une direction sensiblement perpendiculaire à la surface d'appui,
- un support de guidage, formant saillie sur la pièce à main dentaire, apte à tenir le tronçon de liaison et dont au moins une partie en saillie est une pièce rapportée, fixée sur la pièce à main dentaire de façon détachable,
dans laquelle :
- les moyens de fixation comprennent une cavité femelle pratiquée dans la pièce à main dentaire,
- les moyens de fixation comprennent une excroissance mâle, solidaire des moyens de butée, conformée pour pénétrer et être retenue dans la cavité femelle,
- la pièce à main dentaire comporte des moyens de verrouillage, qui interdisent sélectivement la séparation de l'excroissance mâle et de la cavité femelle, et qui sont actionnés par le seul mouvement d'assemblage de la tête sur le corps.

On permet ainsi au praticien plusieurs modes d'utilisation qu'il pourra choisir à volonté : soit il adaptera les moyens de butée pour utiliser un foret avec ou sans épaulement de butée, soit il diminuera l'encombrement de la pièce à main dentaire en retirant une partie au moins des moyens de butée lorsqu'il n'en a pas besoin, pour utiliser alors un foret à épaulement de butée, ou un guide chirurgical à canons de perçage avec un foret dépourvu d'épaulement de butée.

Pour détacher les moyens de butée, il est nécessaire de séparer la tête du corps de la pièce à main dentaire. Une telle séparation est impossible à réaliser lors du fonctionnement de la pièce à main dentaire. Il n'y a ainsi aucun risque de démontage intempestif et accidentel des moyens de butée lorsque le praticien opère le patient.

De préférence, le support de guidage peut être entièrement détachable de la pièce à main dentaire, afin de retirer tout élément formant saillie sur la pièce à main dentaire. On donne ainsi un encombrement minimum à la pièce à main dentaire afin de ne pas gêner le praticien lorsque celui-ci n'a pas besoin des moyens de butée.

Avantageusement, la cavité femelle est pratiquée au voisinage de la tête et est conformée pour permettre l'engagement de l'excroissance mâle selon une direction d'engagement sensiblement perpendiculaire à la direction de pénétration, et pour interdire toute extraction de l'excroissance mâle selon toute autre direction que la direction d'engagement.

De tels moyens de fixation autorisent le montage et la fixation des moyens de butée sur la pièce à main dentaire par un simple mouvement rapide de translation, et sans avoir recours à des outils.

Selon un mode de réalisation préféré de l'invention, on peut prévoir que :
- la tête est fixée de façon amovible à l'extrémité distale du corps par engagement axial dans l'extrémité distale du corps,
- la cavité femelle est pratiquée sur la tête, orientée axialement selon la direction d'engagement et ouverte à son extrémité proximale,
- les moyens de verrouillage comprennent une facette de butée; disposée à l'extrémité distale du corps, qui obture l'extrémité proximale de la cavité femelle une fois la tête assemblée sur le corps,
- l'excroissance mâle des moyens de fixation se développe selon une longueur égale ou peu inférieure à la longueur de la cavité femelle.

La retenue de l'excroissance mâle dans la cavité femelle se fait ainsi par la seule connexion de la tête à l'extrémité distale de corps, sans nécessiter aucune autre pièce. Les moyens de verrouillage et les moyens de fixation sont très simples, ne comportent que peu de pièces et sont en outre réalisables de façon économique sur des pièces à main dentaires existantes sans nécessiter d'adaptation particulière.

De préférence, on peut prévoir que la cavité femelle est une rainure et l'excroissance mâle est une nervure.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue en perspective d'une pièce à main dentaire selon un mode de réalisation de invention, avec un foret dépourvu d'épaulement de butée ;
- la figure 2 est une vue en perspective de la pièce à main dentaire de la figure 1, dans le cas de l'utilisation d'un foret à épaulement de butée ;
- la figure 3 est une vue en coupe transversale de la pièce à main dentaire de la figure 1 ;
- la figure 4 est une vue éclatée de différents éléments des moyens de butée de la pièce à main dentaire de la figure 1 ;
- la figure 5 est une vue en perspective d'un élément des moyens de butée selon l'invention ;
- la figure 6 est une vue de dessus d'un élément des moyens de butée selon l'invention ; et
- la figure 7 est une vue en perspective des différents éléments constitutifs de la pièce à main dentaire selon l'invention.

### DESCRIPTION DES MODES DE REALISATION PREFERES

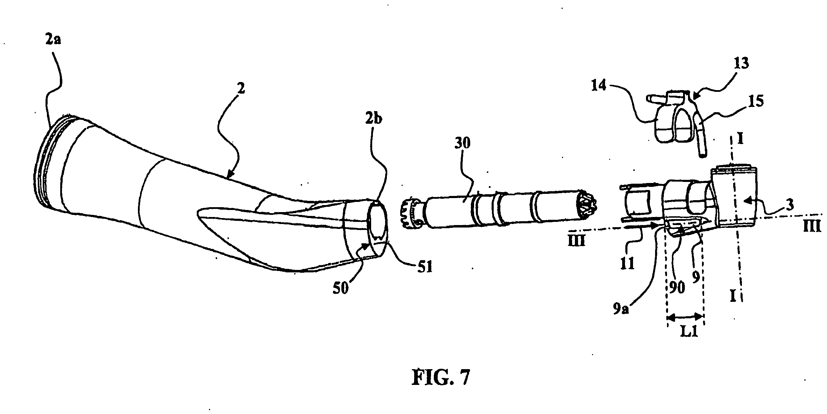

De façon courante, et comme représenté sur la figure 7, la pièce à main dentaire comporte un corps 2 avec une extrémité proximale 2a et une extrémité distale 2b, et une tête 3 démontable destinée à être connectée à l'extrémité distale 2b de corps, apte à tenir un foret.

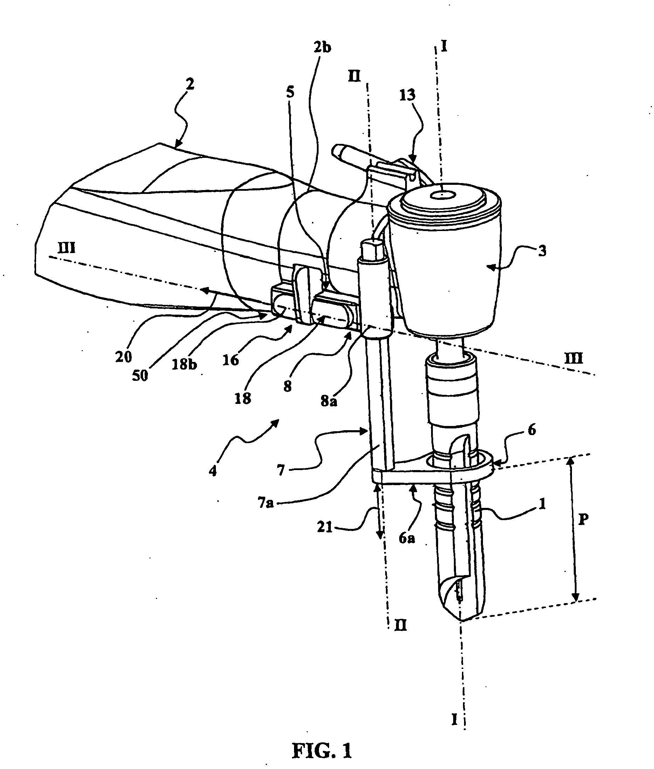

Sur la figure 1, la pièce à main dentaire porte un foret 1 dépourvu d'épaulement de butée. La pièce à main dentaire peut entraîner le foret 1 en mouvement relatif selon une direction de pénétration I-I. La pièce à main dentaire comporte des moyens de butée 4, fixés sur la pièce à main dentaire par des moyens de fixation 5, et permettant de limiter la profondeur de pénétration du foret 1 dans le corps d'un patient.

Les moyens de butée 4 comprennent :
- un corps de butée 6, à surface d'appui 6a apte à prendre appui contre le corps du patient en fin de pénétration du foret 1 dans le corps du patient,
- un tronçon de liaison 7, solidaire du corps de butée 6 et s'étendant selon une direction II-II sensiblement perpendiculaire à la surface d'appui 6a,
- un support de guidage 8, formant saillie sur la pièce à main dentaire, apte à tenir le tronçon de liaison 7. Au moins une partie en saillie du support de guidage 8 est une pièce rapportée, fixée sur la pièce à main dentaire de façon détachable.

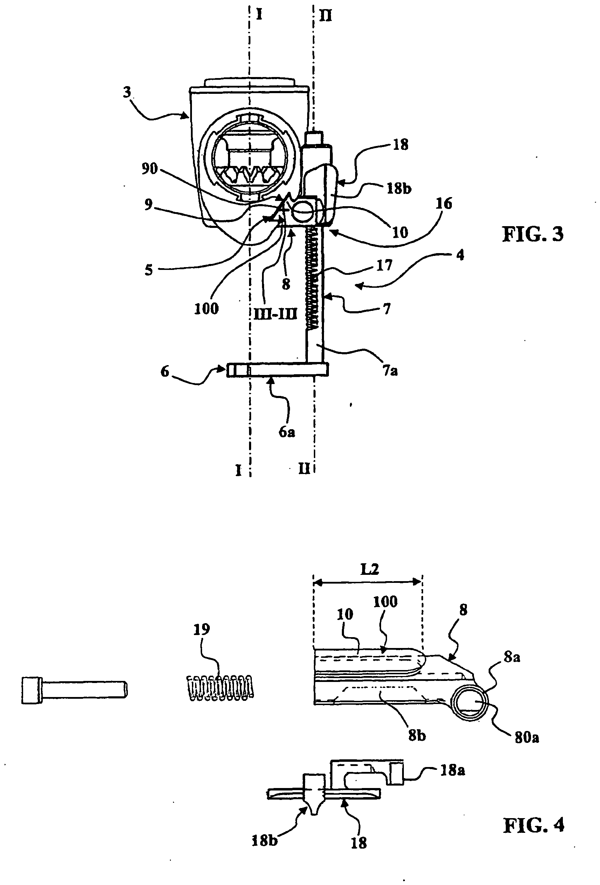

Dans le mode de réalisation qui est illustré, on voit plus particulièrement sur la figure 3 que le support de guidage 8 est entièrement détachable de la pièce à main dentaire, ce qui permet de réduire au maximum l'encombrement de la pièce à main dentaire lorsque les moyens de butée 4 sont inutiles.

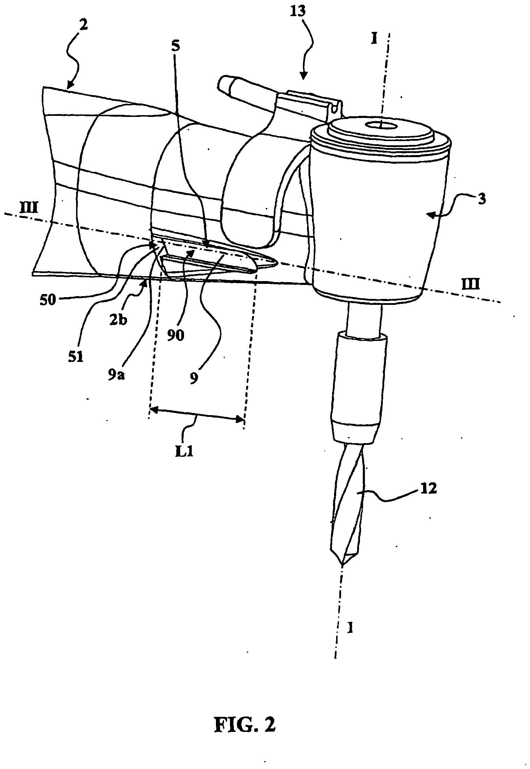

Les moyens de fixation 5 des moyens de butée 4 sur la pièce à main dentaire comprennent une cavité femelle 90 pratiquée dans la pièce à main dentaire au voisinage de la tête 3 (figure 2). Les moyens de fixation 5 comprennent également une excroissance mâle 100 (figures 3 et 4), solidaire des moyens de butée 4, conformée pour pénétrer et être retenue dans la cavité femelle 90.

La pièce à main dentaire comporte des moyens de verrouillage 50, qui interdisent sélectivement la séparation de l'excroissance mâle 100 et de la cavité femelle 90, et qui sont actionnés par le seul mouvement d'assemblage de la tête 3 sur le corps 2.

Les moyens de verrouillage 50 sont simples de conception et d'utilisation et sont nécessaires et suffisants pour empêcher tout démontage intempestif et accidentel des moyens de butée 4 lorsque le praticien utilise sa pièce à main dentaire pour opérer un patient.

La cavité femelle 90 est conformée pour permettre l'engagement de l'excroissance mâle 100 selon une direction d'engagement III-III sensiblement perpendiculaire à la direction de pénétration I-I, et pour interdire toute extraction de l'excroissance mâle 100 selon toute autre direction que la direction d'engagement III-III.

De tels moyens de fixation 5 permettent de monter le support 8 des moyens de butée 4 par un simple mouvement de translation axiale illustré par la flèche 11 sur la figure 7. Le montage des moyens de butée 4 sur la pièce à main dentaire peut donc être effectué par le praticien, de façon simple et rapide et sans avoir recours à des outils.

Comme on le voit plus particulièrement sur la figure 7, la tête 3 est démontable, et peut être fixée de façon amovible à l'extrémité distale 2b du corps 2 par engagement axial dans l'extrémité distale 2b du corps 2. L'arbre de transmission 30 est logé en partie dans le corps 2 et en partie dans la tête 3, de façon connue. La cavité femelle 90 est pratiquée sur la tête 3, selon une longueur L1, orientée axialement selon la direction d'engagement III-I11 et ouverte à son extrémité proximale 9a.

Les moyens de verrouillage 50 comprennent une facette de butée 51, disposée à l'extrémité distale 2b du corps 2.

Une fois la tête 3 assemblée sur le corps 2, les moyens de verrouillage 50 (facette de butée 51) obturent l'extrémité proximale 9a de la cavité femelle 90 (figure 2), empêchant ainsi tout risque de sortie de l'excroissance mâle 100 hors de la cavité femelle 90.

Les moyens de verrouillage 50 sont actionnés par le seul mouvement nécessaire et suffisant d'assemblage de la tête 3 sur le corps 2.

Dans un autre mode de réalisation (non représenté) de l'invention, on prévoit que la cavité femelle 90 est réalisée à la fois en partie dans la tête 3 et dans le corps 2, l'assemblage de la tête 3 et du corps 2 formant une cavité femelle 90 fermée apte à retenir l'excroissance mâle 100. L'excroissance mâle est alors retenue simultanément dans la tête 3 et dans le corps 2.

Dans le mode de réalisation particulier illustré sur les figures 1 à 7, la cavité femelle 90 et l'excroissance mâle 100 sont une rainure 9 et une nervure 10 qui ont des sections transversales trapézoïdales de formes complémentaires permettant d'effectuer un assemblage en queue d'aronde.

Les sections transversales trapézoïdales de formes complémentaires de la rainure 9 et de la nervure 10 permettent d'immobiliser de façon fiable les moyens de butée 4 par rapport à la pièce à main dentaire.

La nervure 10 (figure 4) des moyens de butée 4 se développe selon une longueur L2 égale ou peu inférieure à la longueur L1 (figure 2) de la rainure 9, ce qui permet d'immobiliser totalement le support de guidage 8 dans la rainure 9 comme illustré sur la figure 1. La rainure 9 se développant selon une direction sensiblement perpendiculaire à la direction de pénétration I-I, et la surface d'appui 6a du corps de butée 6 étant sensiblement perpendiculaire à la direction de pénétration I-I, les efforts se produisant sur les moyens de butée 4 ne sont pas susceptibles de faire bouger en translation selon la direction d'engagement III-III la nervure 10 dans la rainure 9. En effet, les efforts s'exerçant sur les moyens de butée ont sensiblement pour direction la direction de pénétration I-I et sont donc perpendiculaires à la direction d'engagement III-III selon laquelle est orientée la rainure 9. Ces forces ne peuvent ainsi pas travailler pour provoquer un déplacement de la nervure 10 dans la rainure 9.

En outre, l'assemblage en queue d'aronde de la rainure 9 et de la nervure 10 empêche tout mouvement des moyens de butée 4 autre qu'une translation selon la direction axiale d'engagement III-III selon laquelle se développe la rainure 9.

Sur la figure 2, les moyens de butée ont été détachés de la pièce à main dentaire pour permettre l'utilisation sans gêne d'un foret 12 à épaulement de butée. On voit qu'aucun encombrement n'est ajouté à la pièce à main dentaire lorsque les moyens de butée ont été détachés de celle-ci. Une fois les moyens de butée détachés de la pièce à main dentaire, la pièce à main dentaire ne présente aucun élément en saillie au voisinage de sa tête 3.

Le praticien peut ainsi utiliser sans gêne un foret 12 à épaulement de butée, ou un foret 1 dépourvu d'épaulement de butée en combinaison avec un guide chirurgical.

Dans le cas où les moyens de butée 4 ne sont pas utilisés, la rainure 9 est apparente et en contre dépouille du fait de sa section transversale trapézoïdale dans la pièce à main dentaire. Afin d'éviter l'accumulation de particules polluantes dans la rainure 9, il est possible d'introduire dans la rainure 9 un bouchon de forme extérieure sensiblement identique à celle de la nervure 10. Le bouchon protège alors la rainure 9 qui reste ainsi propre et dépourvue de tout élément pouvant empêcher ou compliquer l'introduction ultérieure de la nervure 10 dans la rainure 9 lors du montage des moyens de butée 4 sur la pièce à main dentaire.

Dans le mode de réalisation illustré sur les figures 1 à 7, la pièce à main dentaire comporte des moyens de projection de fluide 13. Les moyens de projection de fluide 13 comportent un cavalier de pulvérisation 14 muni d'une canalisation 15 apte à projeter un fluide au voisinage de la zone de travail du foret 1 ou 12 selon au moins un jet de fluide sensiblement parallèle au foret 1 ou 12.

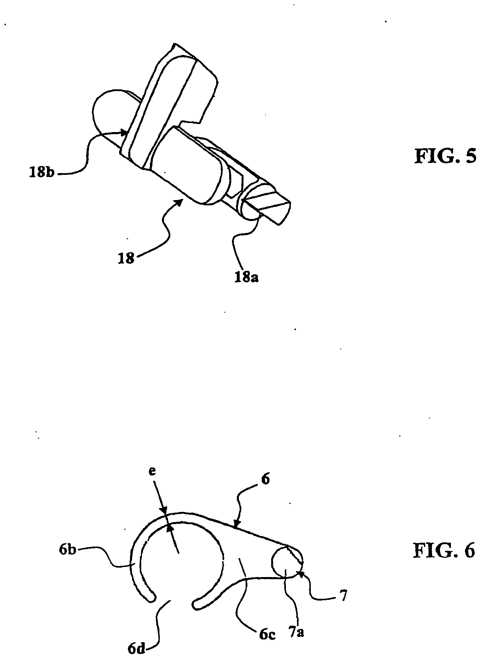

Sur la figure 6, le corps de butée 6 comporte un tronçon distal 6b de forme annulaire et de faible épaisseur e radiale, destiné à entourer le foret 1. Un pont de liaison 6c s'étend depuis le tronçon distal 6b afin de le relier au tronçon de liaison 7.

Le corps de butée 6 comporte une lumière 6d qui se trouve en correspondance avec la trajectoire du jet de fluide (figure 1). On rend ainsi compatible l'utilisation des moyens de butée 4 avec l'utilisation de moyens de projection de fluide pour permettre au praticien de travailler proprement avec une bonne vision de la zone de travail du foret et dans de bonnes conditions d'hygiène.

Dans le mode de réalisation illustré sur les figures 1 à 7, les moyens de butée 4 sont réglables et autorisent plusieurs profondeurs P de pénétration du foret 1 dans le corps d'un patient (figure 1).

Pour ce faire, le tronçon de liaison 7 comporte une tige longitudinale de liaison 7a tandis que le support de guidage 8 comporte un manchon de guidage 8a, dans lequel peut coulisser la tige longitudinale de liaison 7a. Des moyens de blocage 16 permettent d'empêcher toute translation de la tige longitudinale de liaison 7a dans le manchon de guidage 8a.

On voit plus particulièrement sur la figure 3 que les moyens de blocage 16 comportent une pluralité de crans transversaux 17 répartis sur la longueur de la tige longitudinale de liaison 7a. Un coulisseau de blocage 18, représenté plus en détail sur la figure 5, comporte une extrémité distale 18a conformée pour s'engager dans les crans transversaux 17 de la tige longitudinale de liaison 7a.

Comme illustré sur la figure 4, le coulisseau de blocage 18 est destiné à être monté dans un logement 8b du support de guidage 8 pour coulisser latéralement vers et à l'écart de la tige longitudinale de liaison 7a entre une position de blocage dans laquelle son extrémité distale 18a est engagée dans un cran transversal 17 de la tige longitudinale de liaison 7a et une position de libération dans laquelle l'extrémité distale 18a du coulisseau de blocage 18 est à l'écart de la tige longitudinale de liaison 7a.

Afin d'automatiser le blocage en position de la tige longitudinale de liaison 7a dans le manchon de guidage 8a, on prévoit un ressort hélicoïdal 19 pour rappeler en permanence le coulisseau de blocage 18 dans sa position de blocage. Afin de permettre à l'utilisateur de régler la profondeur P de pénétration, le coulisseau de blocage 18 comporte des moyens de préhension 18b par un utilisateur. Comme illustré sur la figure 1, les moyens de préhension 18b permettent à l'utilisateur de manoeuvrer le coulisseau de blocage 18 en translation selon le sens et la direction définis par la flèche 20. On manoeuvre ainsi le coulisseau de blocage 18 depuis sa position de blocage vers sa position de libération à l'encontre de la force de rappel exercée par le ressort hélicoïdal 19. On retire alors l'extrémité distale 18a à l'écart des crans transversaux 17 de la tige longitudinale de liaison 7a qu'il est alors possible de manoeuvrer dans le manchon de guidage 8a selon un mouvement de translation bilatéral illustré par la double flèche 21 afin de régler la profondeur P de pénétration du foret 1.

Si on lâche les moyens de préhension 18b, le coulisseau de blocage 18 est rappelé dans sa position de blocage par le ressort hélicoïdal 19 pour conserver la profondeur de pénétration P choisie.

Afin que le corps de butée 6 ne vienne pas en conflit avec le foret 1, on voit plus particulièrement sur les figures 4 et 6 que le manchon de guidage 8a comporte un alésage interne 80a à section transversale complémentaire en forme de la section transversale de la tige longitudinale de liaison 7a. Les sections transversales de la tige longitudinale de liaison 7a et de l'alésage interne 80a du manchon de guidage 8a empêchent tout mouvement de rotation relatif entre ceux-ci. Par exemple, dans le mode de réalisation illustré sur les figures 1 à 7, la section transversale de la tige longitudinale de liaison 7a est circulaire avec un méplat.

La présente invention n'est pas limitée aux modes de réalisation qui ont été explicitement décrits, mais elle en inclut les diverses variantes et généralisations contenues dans le domaine des revendications ci-après.

## Revendications

1. Pièce à main dentaire permettant de porter un foret (1, 12) et de l'entraîner en mouvement relatif selon une direction de pénétration (I-I), comprenant :
- un corps (2) avec une extrémité proximale (2a) et une extrémité distale (2b) de corps,
- une tête (3) démontable, connectée à l'extrémité distale (2b) de corps, apte à tenir le foret (1, 12),
- des moyens de butée (4), fixés sur la pièce à main dentaire par des moyens de fixation (5), et permettant de limiter la profondeur de pénétration du foret (1, 12) dans le corps d'un patient,
dans laquelle les moyens de butée (4) comprennent :
- un corps de butée (6), à surface d'appui (6a) apte à prendre appui contre le corps du patient en fin de pénétration du foret (1, 12) dans le corps du patient,
- un tronçon de liaison (7), solidaire du corps de butée (6) et s'étendant selon une direction (II-II) sensiblement perpendiculaire à la surface d'appui (6a),
- un support de guidage (8), formant saillie sur la pièce à main dentaire, apte à tenir le tronçon de liaison (7) et dont au moins une partie en saillie est une pièce rapportée, fixée sur la pièce à main dentaire de façon détachable,
**caractérisée en ce que** :
- les moyens de fixation (5) comprennent une cavité femelle (90) pratiquée dans la pièce à main dentaire,
- les moyens de fixation (5) comprennent une excroissance mâle (100), solidaire des moyens de butée (4), conformée pour pénétrer et être retenue dans la cavité femelle (90),
- la pièce à main dentaire comporte des moyens de verrouillage (50), qui interdisent sélectivement la séparation de l'excroissance mâle (100) et de la cavité femelle (90), et qui sont actionnés par le seul mouvement d'assemblage de la tête (3) sur le corps (2).

2. Pièce à main dentaire selon la revendication 1, **caractérisée en ce que** la cavité femelle (90) est pratiquée au voisinage de la tête (3) et est conformée pour permettre l'engagement de l'excroissance mâle (100) selon une direction d'engagement (III-III) sensiblement perpendiculaire à la direction de pénétration (I-I), et pour interdire toute extraction de l'excroissance mâle (100) selon toute autre direction que la direction d'engagement (III-III).

3. Pièce à main dentaire selon l'une des revendications 1 ou 2, **caractérisée en ce que** le support de guidage (8) est entièrement détachable de la pièce à main dentaire.

4. Pièce à main dentaire selon l'une quelconque des revendications 1 à 3, **caractérisée en ce que** :
- la tête (3) est fixée de façon amovible à l'extrémité distale (2b) du corps (2) par engagement axial dans l'extrémité distale (2b) du corps (2),
- la cavité femelle (90) est pratiquée sur la tête (3), orientée axialement selon la direction d'engagement (III-III) et ouverte à son extrémité proximale (9a),
- les moyens de verrouillage (50) comprennent une facette de butée (51), disposée à l'extrémité distale (2b) du corps (2), qui obture l'extrémité proximale (9a) de la cavité femelle (90) une fois la tête (3) assemblée sur le corps (2),
- l'excroissance mâle (100) des moyens de fixation (5) se développe selon une longueur (L2) égale ou peu inférieure à la longueur (L1) de la cavité femelle (90).

5. Pièce à main dentaire selon l'une quelconque des revendications 1 à 4, **caractérisée en ce que** la cavité femelle (90) est une rainure (9) et **en ce que** l'excroissance mâle (100) est une nervure (10).

6. Pièce à main dentaire selon la revendication 5, **caractérisée en ce que** la rainure (9) et la nervure (10) ont des sections transversales trapézoïdales de formes complémentaires permettant d'effectuer un assemblage en queue d'aronde.

7. Pièce à main dentaire selon l'une quelconque des revendications 1 à 6, **caractérisée en ce que** :
- la pièce à main dentaire comporte des moyens de projection de fluide (13) aptes à projeter un fluide au voisinage de la zone de travail du foret (1, 12) selon au moins un jet de fluide sensiblement parallèle au foret (1, 12),
- le corps de butée (6) comporte une lumière (6d) en correspondance avec la trajectoire du jet de fluide.

8. Pièce à main dentaire selon l'une quelconque des revendications 1 à 7, **caractérisée en ce que** :
- le tronçon de liaison (7) comporte une tige longitudinale de liaison (7a),
- le support de guidage (8) comporte un manchon de guidage (8a), dans lequel peut coulisser la tige longitudinale de liaison (7a),
- des moyens de blocage (16) permettent d'empêcher toute translation de la tige longitudinale de liaison (7a) dans le manchon de guidage (8a).

9. Pièce à main dentaire selon la revendication 8, **caractérisée en ce que** :
- le manchon de guidage (8a) comporte un alésage interne (80a) à section transversale complémentaire en forme de la section transversale de la tige longitudinale de liaison (7a),
- les sections transversales de la tige longitudinale de liaison (7a) et de l'alésage interne (80a) du manchon de guidage (8a) empêchent tout mouvement de rotation relatif entre ceux-ci.

10. Pièce à main dentaire selon l'une des revendications 8 ou 9, **caractérisée en ce que** les moyens de blocage (16) comportent :
- une pluralité de crans transversaux (17) répartis sur la longueur de la tige longitudinale de liaison (7a),
- un coulisseau de blocage (18), à extrémité distale (18a) conformée pour s'engager dans les crans transversaux (17) de la tige longitudinale de liaison (7a), apte à coulisser latéralement vers et à l'écart de la tige longitudinale de liaison (7a) dans un logement (8b) du support de guidage (8) entre une position de blocage dans laquelle son extrémité distale (18a) est engagée dans un cran transversal (17) de la tige longitudinale de liaison (7a) et une position de libération dans laquelle l'extrémité distale (18a) du coulisseau de blocage (18) est à l'écart de la tige longitudinale de liaison (7a).

11. Pièce à main dentaire selon la revendication 10, **caractérisée en ce que** :
- un ressort hélicoïdal (19) rappelle en permanence le coulisseau de blocage (18) dans sa position de blocage,
- le coulisseau de blocage (18) comporte des moyens de préhension (18b) par un utilisateur, permettant à celui-ci de manoeuvrer le coulisseau de blocage (18) depuis sa position de blocage vers sa position de libération à l'encontre de la force de rappel exercée par le ressort hélicoïdal (19).

## Claims

1. Dental handpiece for carrying a drill (1, 12) and driving it in relative movement in, a penetration direction (I-I), comprising:
- a body (2) with a proximal body end (2a) and a distal body end (2b),
- a demountable head (3), connected to the distal body end (2b), adapted to hold the drill (1, 12),
- abutment means (4) fixed to the dental handpiece by fixing means (5) to limit the depth of penetration of the drill (1, 12) into the body of a patient,
wherein the abutment means (4) comprise:
- an abutment body (6) with a bearing surface (6a) adapted to bear against the body of the patient at the end of penetration of the drill (1, 12) into the body of the patient,
- a connecting section (7), fastened to the abutment body (6) and extending in a direction (II-II) substantially perpendicular to the bearing surface (6a),
- a guide support (8) forming a projection on the dental handpiece, adapted to hold the connecting section (7), and including at least one projecting part which is an attached part detachably fixed to the dental handpiece,
**characterized in that**:
- the fixing means (5) comprise a female cavity (90) produced in the dental handpiece,
- the fixing means (5) comprise a male protuberance (100), fastened to the abutment means (4), conformed to penetrate and to be retained in the female cavity (90),
- the dental handpiece includes flocking means (50) that selectively prevent separation of the male protuberance (100) and the female cavity (90) and are actuated by the simple movement of assembling the head (3) onto the body (2).

2. Dental handpiece according to claim 1, **characterized in that** the female cavity (90) is produced in the vicinity of the head (3) and is conformed to enable engagement of the male protuberance (100) in an engagement direction (III-III) substantially perpendicular to the penetration direction (I-I) and to prevent all extraction of the male protuberance (100) in any direction other than the engagement direction (III-III).

3. Dental handpiece according to either of claims 1 or 2, **characterized in that** the guide support (8) is entirely detachable from the dental handpiece.

4. Dental handpiece according to any one of claims 1 to 3, **characterized in that**:
- the head (3) is removably fixed to the distal end (2b) of the body (2) by axial engagement in the distal end (2b) of the body (2),
- the female cavity (90) is produced on the head (3), oriented axially in the engagement direction (III-III) and opened at its proximal end (9a),
- the locking means (50) comprise an abutment facet (51), disposed at the distal end (2b) of the body (2), which blocks the proximal end (9a) of the female cavity (90) when the head (3) has been assembled onto the body (2),
- the male protuberance (100) of the fixing means (5) extends over a length (L2) equal to or slightly less than the length (L1) of the female cavity (90).

5. Dental handpiece according to any one of claims 1 to 4, **characterized in that** the female cavity (90) is a groove (9) and **in that** the male protuberance (100) is a rib (10).

6. Dental handpiece according to claim 5, **characterized in that** the groove (9) and the rib (10) have trapezoidal cross sections of complementary shapes allowing to constitute a dovetail joint.

7. Dental handpiece according to any one of claims 1 to 6, **characterized in that**:
- the dental handpiece includes fluid spray means (13) adapted to spray a fluid in the vicinity of the working area of the drill (1, 12) in the form of at least one jet of fluid substantially parallel to the drill (1, 12),
- the abutment body (6) includes an opening (6d) in corresponding relationship to the trajectory of the jet of fluid.

8. Dental handpiece according to any one of claims 1 to 7, **characterized in that**:
- the connecting section (7) includes a longitudinal connecting rod (7a),
- the guide support (8) includes a guide sleeve (8a) in which the longitudinal connecting rod (7a) can slide,
- immobilizing means (16) prevent any translation of the longitudinal connecting rod (7a) in the guide sleeve (8a).

9. Dental handpiece according to claim 8, **characterized in that**:
- the guide sleeve (8a) includes an internal bore (80a) with a cross section of complementary shape to the cross section of the longitudinal connecting rod (7a),
- the cross sections of the longitudinal connecting rod (7a) and the internal bore (80a) of the guide sleeve (8a) prevent all movement in relative rotation between them.

10. Dental handpiece according to either of claims 8 or 9, **characterized in that** the immobilizing means (16) include:
- a plurality of transverse detents (17) distributed over the length of the longitudinal connecting rod (7a),
- an immobilizing slider (18), with a distal end (18a) conformed to engage in the transverse detents (17) of the longitudinal connecting rod (7a), adapted to slide laterally towards and away from the longitudinal connecting rod (7a) in a housing (8b) of the guide support (8) between an immobilizing position in which its distal end (18a) is engaged in a transverse detent (17) of the longitudinal connecting rod (7a) and a release position in which the distal end (18a) of the immobilizing slider (18) is away from the longitudinal connecting rod (7a).

11. Dental handpiece, according to claim 10, **characterized in that**:
- a coil spring (19) urges the immobilizing slider (18) into its immobilizing position at all times,
- the immobilizing slider (18) includes holding means (18b) for a user to hold it by, enabling the user to operate the immobilizing slider (18) from its immobilizing position to its release position against the return force exerted by the coil spring (19).

## Patentansprüche

1. Zahnärztliches Handstück, das es erlaubt, einen Bohrer (1, 12) zu halten und in Bewegung relativ zu einer Richtung des Eindringens (I-I) anzutreiben, enthaltend:
- einen Körper (2) mit einer proximalen Extremität (2a) und einer distalen Extremität (2b) des Körpers,
- einen demontierbaren Kopf (3), der mit der distalen Extremität (2b) des Körpers verbunden ist und ausgebildet ist, den Bohrer (1, 12) zu halten,
- Anschlagmittel (4), die durch Befestigungsmittel (5) am zahnärztlichen Handstück befestigt sind und gestatten, die Eindringtiefe des Bohrers (1, 12) in den Körper eines Patienten zu begrenzen,
wobei die Anschlagmittel (4) enthalten:
- einen Anschlagkörper (6) mit einer Anschlagfläche (6a), die ausgebildet ist, am Ende des Eindringens des Bohrers (1, 12) in den Körper des Patienten gegen den Körper des Patienten in Anschlag zu gelangen,
- ein Verbindungsteil (7), das am Anschlagkörper (6) angebracht ist und sich in einer Richtung (II-II) erstreckt, die im wesentlichen senkrecht zur Anschlagfläche (6a) ist,
- eine Führungshalterung (8), die an dem zahnärztlichen Handstück einen Vorsprung bildet und ausgebildet ist, das Verbindungsteil (7) zu halten und von dem mindestens ein vorspringender Abschnitt ein angesetztes Teil ist, das in lösbarer Weise an dem zahnärztlichen Handstück befestigt ist,
**dadurch gekennzeichnet, dass**:
- die Befestigungsmittel (5) eine Ausnehmung (90) aufweisen, die in dem zahnärztlichen Handstück ausgebildet ist,
- die Befestigungsmittel (5) einen Vorsprung (100) aufweisen, der an den Anschlagmitteln (4) angebracht ist und dazu ausgebildet ist, in die Ausnehmung (90) einzudringen und in ihr gehalten zu werden,
- das zahnärztliche Handstück Mittel zum Verriegeln (50) aufweist, die selektiv eine Trennung des Vorsprunges (100) und der Ausnehmung (90) verbieten und die durch die einzige Montagebewegung des Kopfes (3) gegenüber dem Körper (2) betätigt werden.

2. Zahnärztliches Handstück nach Anspruch 1, **dadurch gekennzeichnet, dass** die Ausnehmung (90) in Nachbarschaft zu dem Kopf (3) ausgebildet ist und dazu eingerichtet ist, einen Eingriff des Vorsprunges (100) in einer Eingriffsrichtung (III-III) zu gestatten, die im wesentlichen senkrecht zur Eindringrichtung (I-I) ist und um jegliches Herausziehen des Vorsprunges (100) in jeder anderen Richtung als der Eingriffsrichtung (III-III) zu verbieten.

3. Zahnärztliches Handstück nach einem der Ansprüche 1 oder 2, **dadurch gekennzeichnet,**
**dass** die Führungshalterung (8) vollständig von dem zahnärztlichen Handstück lösbar ist.

4. Zahnärztliches Handstück nach irgendeinem der Ansprüche 1 bis 3, **dadurch gekennzeichnet,**
**dass**:
- der Kopf (3) in lösbarer Weise an der distalen Extremität (2b) des Körpers (2) befestigt ist, durch axialen Eingriff in die distale Extremität (2b) des Körpers (2),
- die Ausnehmung (90) an dem Kopf (3) ausgebildet ist und axial in der Eingriffsrichtung (III-III) ausgerichtet ist und an ihrer proximalen Extremität (9a) offen ist,
- die Mittel zum Verriegeln (50) eine Anschlagfacette (51) aufweisen, die an der distalen Extremität (2b) des Körpers (2) anbeordnet ist, die die proximale Extremität (9a) der Ausnehmung (90) füllt, wenn der Kopf (3) an den Körper (2) montiert ist,
- der Vorsprung (100) der Fixationsmittel (5) sich längs einer Länge (L2) erstreckt, die gleich oder geringfügig kleiner als die Länge (L1) der Ausnehmung (90) ist.

5. Zahnärztliches Handstück nach irgendeinem der Ansprüche 1 bis 4, **dadurch gekennzeichnet,**
**dass** die Ausnehmung (90) eine Rille (9) ist und dass der Vorsprung (100) eine Rippe (10) ist.

6. Zahnärztliches Handstück nach Anspruch 5, **dadurch gekennzeichnet,**
**dass** die Rille (9) und die Rippe (10) trapezförmige Querschnitte mit komplementären Formen haben, die es erlauben, eine Montage mit einem Schwalbenschwanz auszuführen.

7. Zahnärztliches Handstück nach irgendeinem der Ansprüche 1 bis 6, **dadurch gekennzeichnet,**
**dass**:
- das zahnärztliche Handstück Mittel zum Projektieren von Fluid (13) aufweist, die ausgebildet sind, ein Fluid in der Nähe der Arbeitszone des Bohrers (1, 12) zu projektieren mit mindestens einem Fluidstrahl, der im wesentlichen parallel zu dem Bohrer (1, 12) ist,
- der Anschlagkörper (6) eine Beleuchtung (6d) aufweist, in Korrespondenz mit der Trajectorie des Fluidstrahles.

8. Zahnärztliches Handstück nach irgendeinem der Ansprüche 1 bis 7, **dadurch gekennzeichnet, dass**:
- das Verbindungsteil (7) einen längs verlaufenden Verbindungsschaft (7a) aufweist,
- die Führungshalterung (8) einen Führungsstutzen (8a) aufweist, in welchem der längs verlaufende Verbindungsschaft (7a) gleiten kann,
- die Mittel zum Blockieren (16) es gestatten, jegliche Verschiebung des sich längs erstreckenden Verbindungsschaftes (7a) in dem Führungsstutzen (8a) zu unterbinden.

9. Zahnärztliches Handstück nach Anspruch 8, **dadurch gekennzeichnet, dass**:
- der Führungsstutzen (8a) eine innere Bohrung (80a) mit einem Querschnitt aufweist, der zur Form des Querschnittes des längs verlaufenden Verbindungsschaftes (7a) komplementär ist,
- die Querschnitte des längs verlaufenden Verbindungsschaftes (7a) und der inneren Bohrung (80a) des Führungsstutzens (8a) jegliche relative Drehbewegung zwischen ihnen verhindern.

10. Zahnärztliches Handstück nach einem der Ansprüche 8 oder 9, **dadurch gekennzeichnet, dass** die Mittel zum Blockieren (16) aufweisen:
- eine Vielzahl von quer verlaufenden Rasten (17), die längs der Länge des sich längs erstreckenden Verbindungsschaftes (7a) verteilt sind,
- einen Blockierschieber (18) an der distalen Extremität (18a), der ausgebildet ist, in die quer verlaufenden Rasten (17) des längs verlaufenden Verbindungsschaftes (7a) einzugreifen, und geeignet ist für ein seitliches Gleiten in Richtung und im Abstand zu dem sich längs erstreckenden Verbindungsschaftes (7a) in einem Sitz (8b) der Führungshalterung (8) zwischen einer Position des Blockierens, in welcher seine distale Extremität (18a) in der quer verlaufenden Raste (17) des längs verlaufenden Verbindungsschaftes (7a) in Eingriff steht und in einer Position der Freigabe, in welcher die distale Extremität (18a) des Blockierungsschiebers (18) im Abstand zu dem sich längs erstreckenden Führungsschaft (7a) ist.

11. Zahnärztliches Handstück nach Anspruch 10,
**dadurch gekennzeichnet, dass**:
- eine schraubenförmige Feder (19) den Blockierschieber (18) permanent in seine Position des Blockierens drückt,
- der Blockierungsschieber (18) Greifmittel (18b) für einen Benutzer aufweist, die ihm gestatten, den Blockierschieber (18) aus seiner Position des Blockierens in eine Position der Freigabe zu manövrieren, gegen die Federkraft, die von der schraubenförmigen Feder (19) ausgeübt wird.
